# EUROPEAN PATENT APPLICATION

(11) **EP 1 975 238 A1**
(43) Date of publication of application: **01.10.2008**
(21) Application number: 07704720.7
(22) Date of filing: 10.01.2007
(51) Int. Cl.: C12N 15/85, A61K 31/713, A61P 31/00

(54) **GENE CONSTRUCT, VECTOR AND DNA VACCINE FOR THE VACCINATION OF AQUATIC ANIMALS**

(30) Priority: 11.01.2006 ES 200600053
(71) Applicant: Instituto Nacional De Investigacion y Tecnologia agraria y Alimentaria (INIA), 28040 Madrid (ES)
(72) Inventor: COLL MORALES, Julio, E-28040 Madrid (ES)
(74) Representative: Gonzalez Palmero, Fé
(86) International application number: PCT/ES2007/000006
(87) International publication number: WO 2007/080203

(57) **Abstract**

Improvements introduced in the object of patent application No. P200402093 relative to the immunisation of aquatic animals against infection caused by viral or bacterial pathogens or parasites, using DNA expression systems (vectors) that comprise a 5' enhancer sequence, a promoter and a nucleic acid sequence which encodes an immunogenic peptide of an aquatic animal pathogen. Optionally, the DNA expression system contains elements of a transposon, which, contrary to the vector used in said patent application, increases both the intensity and the duration of expression.

## Description

Improvements introduced in the object of Spanish patent application no. P200402093 for "Gene construct, vector and DNA vaccine for the vaccination of aquatic animals."

### Field of the invention

Improvements introduced in the object of patent application No. P200402093 relative to the immunisation of aquatic animals against infection caused by viral or bacterial pathogens or parasites using DNA expression systems (vectors) which comprise a 5' enhancer sequence, a promoter and a nucleic acid sequence that encodes an immunogenic peptide of an aquatic animal pathogen. Optionally, the DNA expression system is a modification of the 5' enhancer sequence by the inclusion of exogenous sequences, such as those from transposons and other elements, which, contrary to the vector used in said patent application, increase both the intensity and the duration of expression.

### Background of the invention

Spanish patent application P200402093 discloses the use of DNA expression systems which comprise a 5' enhancer sequence, a promoter and a nucleic acid sequence that encodes an immunogenic peptide of an aquatic animal pathogen. In addition, the invention discloses the administration of said DNA expression systems to said aquatic animals.

This certificate of addition proposes some improvements on the object of main patent P200402093. These improvements relate to the use of 5' enhancer sequences by the inclusion of exogenous sequences such as those from transposons and other elements to be used as vectors for DNA vaccination in fish.

Transposons are DNA sequences found in the genomes of many living species which have the capacity to split off from the genome, jump and become integrated in a new location (Tafalla et al., 2005a; Tafalla et al., 2005b). They have enhancer elements that may be used as components of the 5' enhancer region.

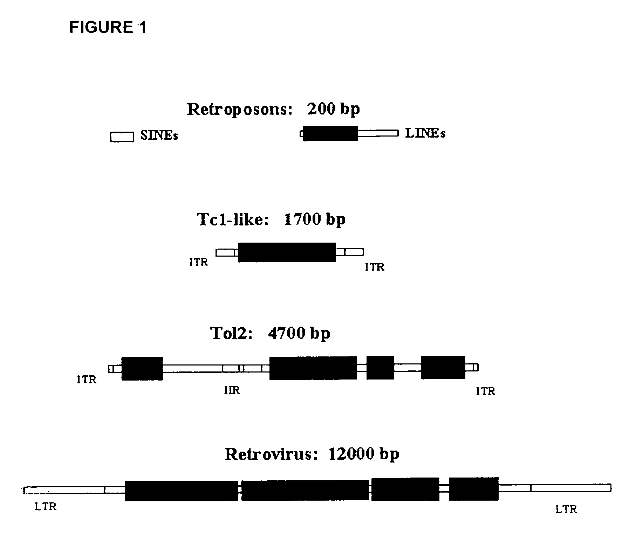

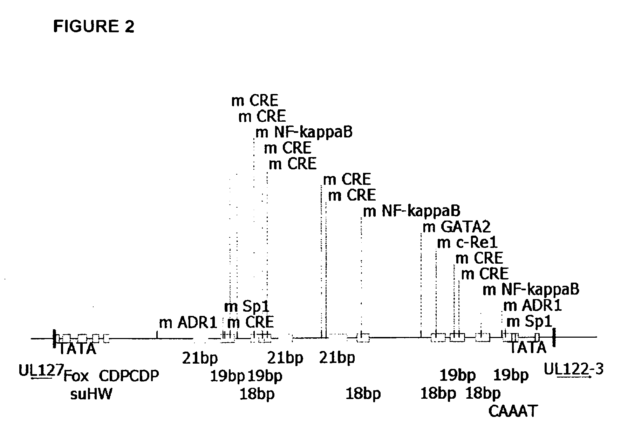

Active transposons are DNA sequences with thousands of bases that contain an enzyme, or transposase, for the mobilisation thereof and two small repeated sequences with hundreds of bases which flank transposase (inverted terminal repeats, ITRs, or long terminal repeats, LTRs). Figure 1 shows a diagram of several types of transposons (retroposons, Tc1-like, To12, and retroviruses) that have been described in fish. In turn, the ITRs (~200 bp) and LTRs (~600 bp) contain numerous 8-12-bp elements grouped in 18-22-bp repeats. Figure 2 shows a diagram of some of the elements that make up the long CMV promoter. These elements are protein-binding sequences that enhance promoter activity or transcription factors. All these sequences may be used as 5' enhancer elements for the promoter.

Finding transposases and complete ITRs /LTRs amongst the thousands of copies that exist in the genome of vertebrates (including fish) is very difficult due to the collection of incomplete or defective copies that form the majority thereof. Although most of the transposons described in fish are also inactivated, there are hypothetically active complete copies in Tc1-like transposons in flatfish (Tpp1, Pleuronectes platessa) (Leaver, 2001) and in trout of the Salvelinus genus (Tsn1, Salvelinus namaycush) (Reed, 1999). Active transposition has even been proven in zebrafish (Tc1-like TE: Tzfl, zebrafish) (Lam et al., 1996) and in medaka (hAT-like TE: To12, Oryzias latipes) (Koga et al., 1996) (Table 1). Amongst retroviruses, some active ones have been described, such as the WDSV Walleye dermal sarcoma virus (Zhang et al., 1999), but it seems that most are inactive, such as the ZFREV zebrafish endogenous retrovirus (Shen et al., 2004).

**Table 1 . Main fish transposons**

| Type | Name | Fish group | tam. *bp | tr. *bp | No. copies per haploid genome | active | GenBank number | reference |
|---|---|---|---|---|---|---|---|---|
| Retro | SmaI | *Oncorhynchusp* | 200 | 7 | 10000- | ? | D90289- | (Kido et al., 1994) |
| | | | | DTR | 100000 | | D90300 | |
| | FokI | *Salvelinus sp* | 200 | 7 | 10000- | ? | D90289- | (Kido et al., 1994) |
| | | | | DTR | 100000 | | D90300 | |
| | HpaI | *Salmonidae* | 200 | 7 | 10000- | ? | D32145- | (Kido et al., 1994) |
| | | | | DTR | 100000 | | D32164 | |
| | AFC | *Cichlids* | 300 | - | 2000- | ? | AB009699- | (TakaHashi et al., 1998) |
| | | | | | 10000 | | AB009714 | |
| | RomI | *Oncorhynchus mykiss* | 600 | 20 DTR | 4000 | ? | | (Winkfein et al., 1988) |
| Tc1 | Tip1 | *Ictalurus punctatus* | 1135 | 85 | ? | ? | X52617 | (Henikoff, 1992) |
| | | | | ITR | | | | |
| | Tes1 | *Eptatretis stouti* | 1499 | 66 | ? | ? | M93037- | (Heierhorst et al., 1992) |
| | | | | ITR | | | M93040 | |
| | Ssal1 SALT | *Salmo salar* | 1535 | 35 ITR | 15000 | no | L22865 | (Goodier and Davidson, 1994) |
| | Tell | *Esox lucius* | 1009 | 223 ITR | ? | no | L41172 | (Ivics et al., 1996a) |
| | Tdr1 | *Danio rerio* | 1205 | 208 | 1000 | no | L12210 | (Radice et al., 1994) |
| | | | | ITR | | | L33472, | |
| | | | | | | | L33469 | (Izsvak et al., 1995) |
| | Tdr2 | *Danio rerio* | 1100 | 100 | 1000 | no | AJ242983 | (Gottgens et al., 1999) (Ivics et al., 1996^{a}) |
| | | | | ITR | | | L48874 | |
| | Tcc1 | *Cyprinus carpio* | 866 | 209 | ? | no | L48683 | (Ivics et al., 1996^{a}) |
| | | | | ITR | | | | |
| | Tcch1 | *Chionodraco hamatus* | 802 | 80 | ? | no | AF305833 | (Capriglione et al., 2002) |
| | | | | | | | AY008266 | |
| | Tom1 | *Oncorhynchus mykiss* | 1689 | 221 | ? | no | L12209 | (Radice et al., 1994) |
| | | | | ITR | | | | |
| | Tss1 | *Salmo salar* | 1619 | 226 | ? | no | L12206- | (Radice et al., 1994) |
| | Tss2 | | | ITR | | | L12208 | |
| | | | | | | | L22865 | (Ivics et al., 1996^{a}) |
| | **Tpp1** | ***Pleuronectes*** | **1627** | **200** | **150** | **yes?** | **AJ249083,** | **(Leaver, 2001)** |
| | PPTN | ***platessa*** | | **ITR** | | | **AJ249085, etc.** | |
| | **Tsn1** | ***Salvelinus namaycush*** | **1643** | **225** | **many** | **yes?** | **AF017232-** | **(Reed, 1999)** |
| | | | | **ITR** | | | **34** | |
| | **SB10** | ***Salmo salar* + *Oncorhynchus mykiss*** | **1639** | **225** | **synthetic** | **yes** | | **(Ivics et al., 1997)** |
| | | | | **ITR** | | | | |
| | | | | | | | | |
| | | | | | | | | |
| | **Tzf1** | ***ZebraFish Danio rerio*** | **1600** | **200** | **700** | **yes** | **U51226-** | **(Lam et al., 1996)** |
| | | | | **ITR** | | | **U51230** | |
| hAT | **To12** | ***Oryzias*** | **4700** | **12** | **10-30** | **yes** | **D84375** | **(Koga et al., 1996)** |
| | | ***latipes*** | | **ITR** | | | | |
| retro | **WDSV** | ***Stizostedion vitreum*** | **12000** | **590** | **1** | **yes** | **AF033822** | **(Zhang et** |
| virus | | | | **LTR** | | | | **al., 1999)** |
| | ZFERV | *ZebraFish Danio rerio* | 12000 | 694 | 1 | ? | AF503912 | (Shen et al., 2004) |
| | | | | LTR | | | | |

| | | | | | | | | |
|---|---|---|---|---|---|---|---|---|
| .T, TE. TR, terminal repeats. ITR, inverted tr. DTR, direct TR. LTR, long terminal repeats. .* bp, sizes in approximate base pairs .underlined, the initials taken for the transposon nomenclature .bold, active fish transposons | | | | | | | | |

The greatest use of active transposases has been studied in mammalian cells: human, from mice and in model fish such as zebrafish and medaka.

Two active fish transposons have been studied in detail: SB, which was "resuscitated" by genetic engineering (Ivics et al., 1997), and Tol2, which was isolated from an inserted gene by the active transposon (Iida et al., 2004; Kawakami et al., 2004; Kawakami and Shima, 1999). Two retroviruses have been described in fish: WDSV [Zhang, 1999 #3394] and ZFREV [Shen, 2004 #2813].

The active "Sleeping Beauty", SB, salmon transposon was reconstructed by means of directed mutagenesis (about 40 mutations for the first version of SB10).

The SB transposon is functional in fish cells (Izsvak et al., 2000), in the cells of mice and humans (Dupuy et al., 2002), and is capable of long-term expression (Yant et al., 2000) in transgenic animals such as mice (Dupuy et al., 2002; Horie et al., 2003; Masuda et al., 2004; Yusa et al., 2004), medaka (Grabher et al., 2003) and zebrafish (Davidson et al., 2003). Under controlled conditions, there may be only one insertion per target molecule, but over 1,000 per genome may occur (Ivics et al., 1997; Izsvak et al., 1997).

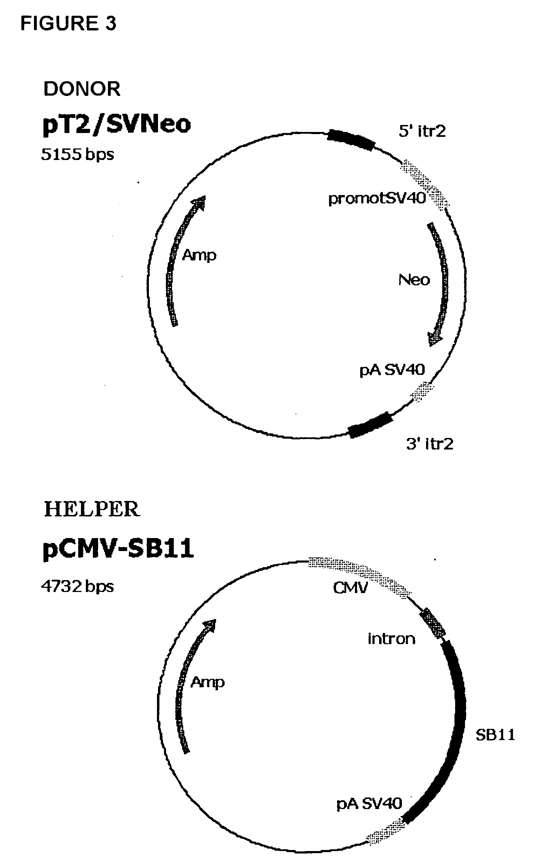

The SB transposition requires two 25-30-pb binding sites inside the ITRs (Izsvak et al., 2000; Izsvak et al., 2002) and cellular factors (Zayed et al., 2003), in order to cut the DNA sequences between the ITRs and splice them in the genome between two TA sites (Plasterk et al., 1999). Figure 3 shows a diagram of the most generalised use of the SB transposon. To do so, it has been divided into 2 independent plasmids: the Donor plasmid, which carries the ITRs, and the Helper plasmid, which carries transposase. The co-transfection of both plasmids makes it possible for the sequences that are between the ITRs in the Donor plasmid to become incorporated into the host cell genome.

In SB transposase, there are nuclear location signals (NLS) in aa 105-120 and PAI-RED regions of α helices (leucine-zipper) in aa 1-110 to bind DNA (Ivics et al., 1996b; Yant et al., 2004). A third domain is the catalytic one or DDE motif (aa 146-290) (Plasterk et al., 1999). The transposition does not seem to be inhibited by the increase in SB expression (Izsvak et al., 2000).

The size limit of the sequence between the ITRs does not seem to be as critical as in the LyR of retroviruses. However, with every Kbp of size increase in the SB transposon insert (from 2.2 to 10.3 Kb), a 30% decrease in the transposition efficiency is obtained (Izsvak et al., 2000), which might be advantageous for the stability thereof.

Between 2002-2004, new mutations in SB10 have been published which increase the transposition efficiency in human cells: SB11 (T136R, M243Q, V253R, A255R) (Geurts et al., 2003), SB12 (R115H, D260K) (Zayed et al., 2004) and HSB3 (K13A, K33A, T83A) (Mikkelsen et al., 2003; Yant et al., 2004). Several ITR versions have also been published: T1 (Izsvak et al., 2000), T2 (Cui et al., 2002) and T3 (Mikkelsen et al., 2003; Yant et al., 2004). On the other hand, vectors have been designed which include pT and SB in a single plasmid (Harris et al., 2002). None of these new versions has been assayed in fish cells.

The original transposase sequence was mutated in 32 positions, leading to the first SB10 active transposase (Ivics et al., 1997).

The "traditional" vectors described in Spanish patent application P200402093 are capable, once the cell membrane has been crossed with the aid of sonication, of expressing the vaccine antigen in the cell cytoplasm, thereby increasing the immunological response to this antigen; however, the expression of said antigen is temporary, occurring only whilst the expression lasts. Consequently, there is a need for more efficient vectors for application in DNA vaccines against fish rhabdovirosis.

The improvement provided by said certificate of addition is the use of transposons or the ITRs/IIRs/LTRs thereof as vectors or parts of vectors for DNA vaccination, since they become integrated in the cell genome and, consequently, the expression of the vaccine antigen is permanent during the lifetime of the animal thus immunised. Moreover, other improvements of the 5' enhancer region have been achieved by the inclusion of elements from the transposon ITRs /IIRs /LTRs, as well as the deletion of an intron.

### Synopsis of the invention

Spanish patent application P200402093 provides, in general, methods and compositions designed to immunise aquatic animals against infection caused by viral or bacterial pathogens or parasites. Basically, the nucleic acid sequence that encodes an immunogenic peptide of an aquatic animal pathogen is introduced into said animal; consequently, said immunogenic peptide is expressed in the animal cells, thereby inducing an immune response that confers protection against the infection caused by said pathogen.

Therefore, in one aspect, patent application P200402093 is related to a gene construct, hereinafter gene construct of patent application P200402093, which comprises an expression control sequence capable of directing the expression of an immunogenic peptide in an aquatic animal, and a nucleic acid sequence that encodes an immunogenic peptide of an aquatic animal pathogen. Where said expression control sequence is composed, in turn, of 2 sequences: a nucleotide sequence that comprises an A sequence and a B sequence, where said A sequence comprises an expression enhancer sequence, and said B sequence comprises a promoter sequence, and where the 3' end of said A sequence is bound to the 5' end of said B sequence.

Occasionally in this description, the expression control sequence present in the gene construct of patent application P200402093, which comprises said A and B sequences, is called "long CMV promoter", in order to distinguish it from other control sequences that comprise the CMV promoter but lack said expression enhancer sequence (A sequence) in 5' with respect to the CMV promoter. This expression control sequence, which comprises only the CMV promoter, and is beyond the scope of the invention, is occasionally called "short CMV promoter" in this description.

The A sequence comprises an expression enhancer sequence. The length of said A sequence may vary within a wide interval, for example, between 100 and 1,000 bases, although in a particular embodiment said A sequence is 218 bases long. Practically any sequence that is capable of enhancing the expression of a peptide in an aquatic animal could be used in the gene construct of the invention; however, in a particular embodiment, said A sequence comprises, or is composed of, the 218-base sequence from nucleotide 1 to nucleotide 218 of the nucleotide sequence shown in SEQ ID NO:1.

The B sequence comprises, or is composed of, the short CMV promoter sequence. Said sequence is already known and is found in commercial plasmids, for example, the pcDNA1Amp plasmid (Invitrogen).

In a practical embodiment, the expression control sequence present in the gene construct of patent application P200402093 is formed by said A and B sequences and comprises, or is composed of, the 798-nucleotide sequence shown in SEQ ID NO:1.

The gene construct of patent application P200402093 comprises, operatively bound to said expression control sequence capable of directing the expression of an immunogenic peptide in an aquatic animal, a nucleic acid sequence that encodes an immunogenic peptide of an aquatic animal pathogen. As used in this description, the expression "operatively bound" means that the immunogenic peptide is expressed within the correct reading frame under the control of said expression control sequence.

"Aquatic animal", as used here, includes any multicellular organism that lives in water, typically fish. Preferably, said aquatic animal is an animal that belongs to a fish species cultured by means of aquaculture. Illustrative examples of said aquatic animals include teleost fish, such as vertebrate fish, for example, salmonids (e.g. trout, salmon), carp, turbot, gilthead sea bream, sea bass, etc.

The term "immunogenic peptide of an aquatic animal pathogen", as used here, refers to complete proteins and fragments thereof, as well as peptides that are epitopes (for example, CTL epitopes), associated with viruses, bacteria or parasites that cause disease in aquatic animals. Therefore, the nucleic acid sequence that encodes an immunogenic peptide present in the gene construct of patent application P200402093 encodes an immunogenic peptide derived from a viral or bacterial pathogen, or a parasite, of an aquatic animal.

When humoral immunity is desired, the nucleic acid sequence that encodes the immunogenic peptide will be, advantageously, a nucleic acid sequence that encodes the complete antigenic protein, or a relatively large fragment thereof, instead of nucleic acid sequences that encode small fragments. However, when cellular immunity is desired, or when humoral immunity may be harmful to the aquatic animal, the nucleic acid sequence that encodes the immunogenic peptide will be, advantageously, a nucleic acid sequence that encodes a relatively small fragment of the corresponding antigenic protein (CTL epitopes).

In a particular embodiment, the gene construct of patent application P200402093 comprises a nucleic acid sequence that encodes an immunogenic peptide of a viral pathogen, such as novirhabdovirus, in an aquatic animal. As an illustration, said peptide may be protein G (pG) or the nucleoprotein (N) of the viral haemorrhagic septicaemia virus of trout (VHSV); the pG or Np of the infectious haematopoietic necrosis virus (IHNV); proteins VP1, VP2, VP3 or the structural Np of the infectious pancreatic necrosis virus (IPNV); the pG of spring viremia of carp (SVC); etc.

In another particular embodiment, the gene construct of patent application P200402093 comprises a nucleic acid sequence that encodes an immunogenic peptide of a bacterial pathogen of an aquatic animal, such as some *Renibacterium* proteins, for example, protein p57 of R. *salmoninarum.* Additional illustrative examples of said immunogenic peptides of bacterial pathogens are cited in US 5.780.448.

Likewise, in another particular embodiment, the gene construct of patent application P200402093 comprises a nucleic acid sequence that encodes an immunogenic peptide of a pathogenic parasite of an aquatic animal, such as the surface antigens of *Ichthyophthirius* (US 5.780.448).

The gene construct of patent application P200402093 may contain two or more nucleic acid sequences that encode two or more immunogenic peptides of one or more aquatic animal pathogens. In this case, the expression control sequence present in the gene construct of patent application P200402093 may direct the expression of said nucleic acid sequences that encode said immunogenic peptides provided that the latter are bound in a reading frame to express a fusion protein. This situation may occur, for example, in order to express proteins VP2 and VP3 of IPNV as a fusion protein.

The gene construct of patent application P200402093 may be obtained by using widely known techniques in the state of the art (Sambrook et al., 1989).

The gene construct of patent application P200402093 may be advantageously found in a suitable vector. Therefore, in another aspect, the invention is related to a vector, hereinafter vector of the invention, which comprises, at least, one gene construct of the invention. This vector may be a viral or a non-viral vector. In general, the choice of the vector will depend on the host cell wherein it will be subsequently introduced. The vector of the invention may be obtained by conventional methods known to those skilled in the art (Sambrook et al., 1989).

This certificate of addition provides the same aspects and particular embodiments as patent application P200402093, and provides the following improved aspects with respect to the aspect of main patent P200402093.

One general aspect of this certificate of addition refers to a vector that comprises a gene construct described in main patent application P200402093, where, preferably, said vector contains elements of a fish transposon. This transposon may be used in the preparation of a vaccine intended to confer protection to aquatic animals against aquatic animal pathogens, which constitutes an additional aspect of this invencion.

One particular aspect of this certificate of addition refers to an alteration of the control sequence described in main patent application P200402093, wherefrom an intron of the cytomegalovirus promoter has been deleted. This vector may be used in the preparation of a vaccine designed to confer protection to aquatic animals against aquatic animal pathogens, by means of a process that comprises the immersion of said aquatic animals in a bath containing said vaccine and the sonication of said bath containing said aquatic animals and said vaccine.

One particular aspect of this certificate of addition refers to a vaccine that comprises an alteration of the control sequence disclosed in main patent application P200402093, wherefrom the vector intron has been deleted and, optionally, comprises one or more pharmaceutically acceptable adjuvants and/or vehicles. More particularly, said vaccine is a DNA vaccine. Said vaccine is useful to protect aquatic animals against viral or bacterial infections, or those caused by parasites.

As used in this description, the term "vaccine" refers to a material that is capable of producing an immune response. Therefore, the vaccine of this certificate of addition is capable of immunising aquatic animals against diseases caused by pathogens of said aquatic animals. Moreover, as is well-known, the activation of CTL (cytotoxic T lymphocytes) activity resulting from the *in vivo* synthesis of the immunogenic peptide could produce immunity against said diseases, not only from a prophylactic standpoint, but also from a therapeutic standpoint; specifically, following the development of the disease in aquatic animals kept in aquaculture facilities.

The vaccine of this certificate of addition is useful to protect aquatic animals susceptible to being infected by aquatic animal pathogens. In a particular embodiment, said pathogens are pathogens of aquatic animals cultured in aquaculture facilities. Non-limiting examples of said pathogens include VHSV, VHNV, IPNV, the spring viremia of carp virus (SVCV), *Aeromonis salmonicida, Renibacterium salmoninarum, Yersinia, Pasteurella* (including *piscicida*), *Vibrosis* (including *anguillarum* and *ordalii*), *Edwardsiella* (including *ictaluri* and *tarda*), *Streptococci, Ichthyophthirius,* etc.

The vaccine of this certificate of addition may be prepared in the form of an aqueous solution or suspension, in a pharmaceutically acceptable vehicle, such as saline solution, phosphate-buffer saline solution (PBS) or any other pharmaceutically acceptable vehicle.

In the sense used in this description, the expression "effective quantity" refers to an effective quantity for the treatment and prevention of diseases caused by aquatic animal pathogens.

The pharmaceutically acceptable vehicles that may be used to formulate the vaccine of this certificate of addition must be sterile and physiologically compatible, such as, for example, sterile water, saline solution, aqueous buffers such as PBS, alcohols, polyols and similar. Moreover, said vaccine may contain other additives, such as adjuvants, stabilisers, antioxidants, preservatives and similar. The available adjuvants include, but are not limited thereto, aluminium salts or gels, carbomers, non-ionic block copolymers, tocopherols, muramyl dipeptide, oily emulsions, cytokines, etc. The quantity of adjuvant to be added depends on the adjuvant's own nature. The stabilisers that are available to be used in vaccines are, for example, carbohydrates, including sorbitol, manitol, dextrine, glucose, proteins such as albumin and casein, and buffers such as alkaline phosphates. The preservatives available include, amongst others, thimerosal, merthiolate and gentamycin.

The vaccine of this certificate of addition may be administered by any appropriate administration route which leads to an immune response that protects against the pathogen in question; to this end, said vaccine will be formulated in the suitable form for the chosen administration route. Although the vaccine of this certificate of addition may be administered by oral route, intramuscular route, by particle bombardment or in spray, by conventional methods (US 5,780,448), when simultaneously immunising a large number of aquatic animals, it is preferable to submerge said aquatic animals in a solution comprising the vaccine of the invention. To this end, the vaccine of this certificate of addition may be prepared in the form of an aqueous solution or suspension, in a pharmaceutically acceptable vehicle, such as saline solution, phosphate-buffer saline solution (PBS) or any other pharmaceutically acceptable vehicle.

The vaccine of this certificate of addition may be prepared using conventional methods known by persons skilled in the art. In a particular embodiment, said vaccine is prepared by mixing, if applicable, the transposon of the invention with, optionally, one or more pharmaceutically acceptable adjuvants and/or vehicles.

Various assays performed by the inventors have shown that very good results are obtained when the aquatic animals to be immunised are subject to an immersion process in a bath that comprises a vaccine of this certificate of addition and the application of ultrasound to said bath containing said vaccine and said aquatic animals. The application of ultrasound may be performed simultaneously with or subsequent to the immersion of said aquatic animals in said bath. Although we do not wish to be bound by any theory, it is believed that the application of ultrasound (sonication) opens interstices and/or pores between the cells and tissues and/or in the cell membranes of the epithelia of aquatic animals, thus allowing for the vaccine of this certificate of addition to penetrate into the aquatic animal in a non-traumatic, hardly stressful manner for the aquatic animal.

### Description of the figures

- Figure 1: shows a diagram of the main families of fish transposons. The most studied types of fish transposons belong to the retroposon, Tc1-like, To12 and retrovirus groups. Black rectangles, reverse transcriptase in retroposons, transposase exons and retrovirus proteins. The ITRs, IIRs and LTRs contain enhancer elements.
- Figure 2: shows some of the 8-12-bp elements grouped in 18-22 bp which make up the long CMV promoter. The addition, elimination or substitution of these elements may enhance promoter activity.
- Figure 3: shows an example of the SB/IIR double plasmid system. In order to induce transposition, the cells are transfected with: i) the donor plasmid or ITR construct and ii) the helper plasmid containing the specific transposase for the donor plasmid. In order to assay the transposition, the transfected cells are put in the presence of the antibiotic whose resistance gene has been placed in the ITR construct (in this example, G418) (Izsvak et al., 2000). Black rectangles, ITRs and SB transposases. Arrows, antibiotic resistance genes. CMV and promotSV40, promoters. pA, transcription termination signal of the SV40 virus.

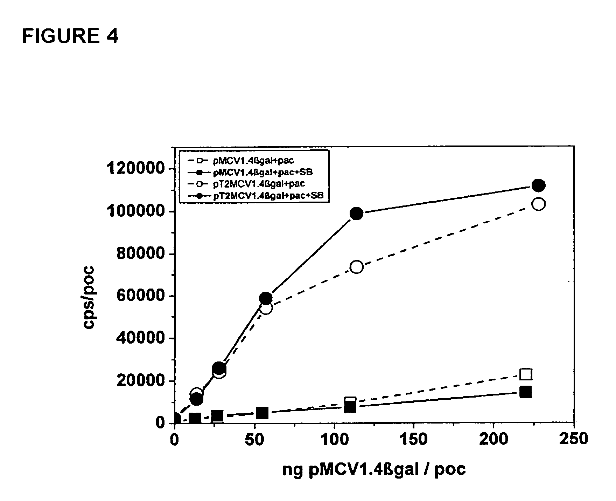

- Figure 4: shows the high activity of β-galactosidase in carp EPC cells when said gene is transfected between the two ITR signals of the SB transposon, compared to the low activity when the same gene is transfected without the ITR signals. By injecting young trout, a two-fold increase in interferon IFN2 and Mx levels has been shown when the G gene of VHSV is injected between two ITRs, as compared to the same gene without ITRs (Table 4).

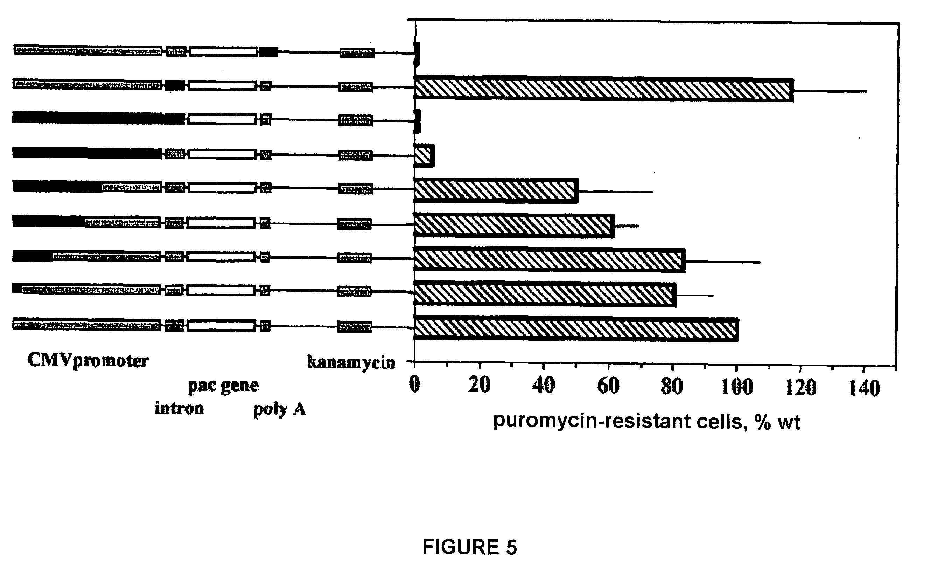

- Figure 5: shows the resistance of EPC cells to puromycin following the transformation of said cells with deleted vectors in different fragments of the 5' enhancer sequence and the control sequence. When the corresponding intron is deleted, a 5%-20% increase in activity takes place. When other enhancer sequences are deleted, greater activity is not obtained.

### Detailed description of the invention

The main object of this certificate of addition is the use of transposons or some of the elements thereof (ITRs, IIRs, LTRs) as vectors or parts of vectors for DNA vaccination in fish.

The methodology that is the object of the invention is based, firstly, on the adaptation of the SB transposon as a vector in fish cells. In order to use, in a practical manner, the SB transposon as a vector, it was separated into two complementary plasmids; one of them carried transposase and the other carried the ITR signals (Figure 3).

On the other hand, although patent application P200402093 used a construct that comprised a nucleic acid sequence which encoded an immunogenic peptide of an aquatic pathogen and an expression control sequence wherein said sequence comprised an expression enhancer sequence and a cytomegalovirus promoter sequence, the construct described above was modified in this certificate of addition.

The modifications introduced in said construct, called pMCV1.4-G, were based on the deletion of the cytomegalovirus promoter intron. Moreover, this new construct was introduced between the two ITR signals, thus obtaining a new vector called pT2pMCV1.4-G. Since fish have endogenous transposases, it was not necessary to have the plasmid containing the exogenous transposase intervene.

Both the transcription of the G gene (Table 2) and the expression of marker proteins such as β-galactosidase (Table 3) significantly increased when using the new vector *in vitro* in transfected cells. The cellular transformation experiments performed with this new construct showed a 10-fold increase when using the pT2pMCV1.4-pac construct as compared to the pMCV1.4-pac construct (Figure 4), and a 10%-20% increase in transformation efficiency when deleting the intron (Figure 5).

### EXAMPLES

The following examples serve to illustrate, but do not limit, this invention.

### Example 1: Adaptation of the SB transposon as a vector in fish cells

### Starting plasmids and constructs.

The starting constructs are the pT2/HB constructs (Material Transfer Agreement Univ. Minnesota) (Cui et al., 2002; Geurts et al., 2003) described in Figure 3.

TA sequences were added to the original ITR sequence (left and right) of the pT/HB construct, which increased the transposition frequency, resulting in pT2 (Cui et al., 2002).

As a starting plasmid, pMVC1.4 (2.8 Kpb) (Ready Vector, Madrid, Spain) was primarily used. It contains an MCS under the control of the early cytomegalovirus (CMV) promoter and a small hybrid intron of the first intron of human β-globin and murine immunoglobin. Now then, whereas most commercial plasmids contain the minimum promoter (<100 pb) and - 400 pb of enhancer sequences, pMVC1.4 additionally contains - 200 pb of enhancer sequences and an intron (long CMV promoter, -737+61+intron). Using this construct, a 3-4 times greater transfection efficiency in EPC has been shown (Rocha et al., 2004). In addition, deletion of the intron increases the efficiency of this promoter in EPC cells by 10%-20% (Figure 5).

### Constructs with the puromycin resistance gene (pac).

It has been demonstrated that EPC cells and zebrafish embryos are ten times more sensitive to puromycin (Sanchez-Puig and Blasco, 2000) than to G418 (unpublished results). EPC was resistant to both hygromycin and zeocin (unpublished results). Therefore, although the first experiments were performed with G418 (by adding 1,000 µg/ml of G418 to the media and using the pTSVNeo and pT2SVNeo constructs), once the puromycin resistance gene (20-50 µg/ml) was transferred to the vectors, these constructs were used in the selection of transformers.

Similar methods to the one described in detail herein were used to subclone the puromycin genes and the long CMV promoters or pG mutants into those plasmids where it was necessary (Fernandez-Alonso et al., 1999; Ferndndez-Alonso et al., 2001). The genes were obtained by means of preparative digestion starting from 2 µg of the starting construct with the suitable restriction endonucleases (GibcoBRL, Postfach, Germany), for 2 h at 37°C. 2 µg of the target plasmid were treated in the same conditions. The digestion product was separated in 1% agarose gel (Sambrook et al., 1989), and the bands obtained were recovered and purified using the SNAP commercial kit (InVitroGen, USA). At least 50 ng/µl of DNA were recovered for each band. The plasmid and the insert were bound with cohesive ends (plasmid:insert ratio 2:3, 1:3 and 1:6), at ambient temperature for 2 h, using T4 DNA ligase (GibcoBRL) over a final volume of 10-20 µl per binding mixture. Controls without inserts were included (Estepa et al., 2001, Estepa et al., 1994). Subsequently, *E*. *coli* Top 10 competent bacteria and/or DH5α commercial bacteria (Electrocompetent Cells, Clontech) were transformed by electroporation. For the electroporation, 10 µl of competent bacteria mixed with 2 µl of binding mixture and 20 µl of water pre-cooled in ice, were electroporated in 0.1-cm cuvettes (Eppendorf). The electroporation conditions were 1.8 KV ~ 0.5 ms. In both cases, following the transformation, 0.3 ml of LB medium (10 g of tryptone, 5 g of yeast extract and 10 g of NaCl per litre, pH 7.5) were added, and the bacteria were incubated at 37°C for 40 min. Subsequently, 5-50 µl were seeded in plates with the selection TB-antibiotic (100 µg/ml) and incubated at 37°C overnight.

Extraction of the plasmid DNA in minipreps was performed from 10 transformed colonies and one binding control colony. To this end, 3-ml cultures of TB-Ampicillin/kanamycin medium (100 µg/ml) were incubated with the colonies in 13-ml tubes (Starstedt, Inc., Newton, USA) at 37°C under shaking for 20 h. The bacterial precipitate was recovered by centrifugation at 12,000 rpm for 2 min and was subject to lysis with STET solution (50 mM Tris-HCl, pH 8; 50 mM EDTA, pH 8; 8% sucrose; 0.1% TritonX100) and lysozyme (1 mg/ml) for 2 min at ambient temperature and for 2 min in a thermal block at 100°C. It was then centrifuged at 12,000 rpm for 8 min and the supernatant was recovered; it was treated with RNase (50 µg/ml) for 30 min at 37°C. The DNA was treated for 5 min at ambient temperature with a 5% CTAB solution (Sigma) (0.2% final), previously solubilised, and centrifuged at 12,000 rpm for 10 min. The recovered precipitate was dissociated from the 5 M CTAB NaCl and the DNA was precipitated with ethanol at -70°C for 20 min and washed with cold 70% ethanol (Estepa et al., 2001; Estepa et al., 1994). The precipitate was re-suspended in sterile distilled H₂O (Sigma).

In order to detect the presence of the insert, the DNA extracted from the different clones was digested. The digestion was performed at 37°C for 2 h. The digestion products were separated in 1% agarose gel and one of the clones containing the subcloned insert in the adequate direction was selected (Fernandez-Alonso et al., 1999; Fernández-Alonso et al., 2001).

### Transposition assays.

Methods previously described for mammalian cells (Cui et al., 2002; Ivics et al., 1997) were used, adapted to EPC cells in our laboratory (López et al., 2001; Rocha et al., 2004). Briefly, EPC monolayers at 0.5 x 10⁶ cells/ml are transformed in 96-well plates with <0.1 µg of transposon vector (pT) and <0.1 µg of transposase vector (SB) in 0.6 µl of Fugene (Rocha et al., 2004). Three days later, culture medium with 1,000 µg/ml of G418 or 20-50 µg/ml of puromycin is added. After 2-3 days, the medium is changed in order to remove the great majority of dead cells and 2-3 days later, the cells are counted, or, 2-3 weeks later, the surviving clones are counted after fixating them in 1% glutaraldehyde and staining them with Giemsa. The results obtained are shown in Table 3.

### Transcription assays.

RNA was obtained using Trizol. Subsequently, the cDNA were synthesised by means of reverse transcriptase and amplified with PCR (RT-PCR), as described in detail in Tafalla et al. (Tafalla et al., 2005). The primers for the amplification of protein G by PCR were previously described (Estepa et al., 1995). The results obtained are shown in Table 2.

### In vivo assays.

20-cm trout were injected with 1 µg of plasmid per trout. Fourteen days later, their livers were isolated and the RNA levels pertaining to IFN2, Mx and MHCII were estimated by RT-PCR using specific primers, as has been recently described (Tafalla et al., 2005). The results obtained are shown in Table 4.

### Results.

The modifications introduced are the introduction of the MCVl.4-gen sequences between two ITRs of pT2 (Figure 3) and the deletion of the intron present in the previous constructs. The genes used include: β-galactosidase, the puromycin resistance gene (pac) and the VHSV G protein gene.

Both the transcription of the G gene (Table 2) and the expression of marker proteins such as β-galactosidase (Table 3) significantly increased when using the new vector in vitro in transfected cells. The cellular transformation experiments performed with this new construct showed a 10-fold increase when using the pT2pMCV1.4-pac construct as compared to the pMCV1.4-pac construct (Figure 4), and a 10%-20% increase in transformation efficiency when deleting the intron (Figure 5). By injecting young trout, a two-fold increase in interferon IFN2 and Mx levels has been shown when the G gene of VHSV is injected between two ITRs, as compared to the same gene without ITRs (Table 4).

**Table 2.**

| | |
|---|---|
| Relative levels of G-encoding mRNA in RTG cells 6 days after being transfected with 0.5 µg of plasmids per well (24 wells per plate) | |
| **Plasmid** | **Relative units** |
| **pMCV1.4 G** | 22,000 ± 15,000 |
| **pT2 pMCV1.4 G** | 350,000 ± 40,000 |

Means ± standard deviations of 3 wells are represented

**Table 3**

| Number of RTG cells 6 days 100 ng of plasmids per well after being transfected with (96 wells per plate) | |
|---|---|
| **plasmids** | **Number of cells stained with Xgal** |
| pMCV1.4 β-gal | 45 ± 1 |
| pT2 MCV1.4 β-gal | 160 ± 8 |

Means ± standard deviations of 3 wells are represented

**Table 4**

| GAPDH-related expression of interleukins in trout liver, 14 days after being injected with 1 µg of plasmid per trout | | | |
|---|---|---|---|
| **Plasmid** | **IFN2** | **Mx** | **MHCII** |
| **Ctrl** | 0.28 ± 0.05 | 0.01 ± 0.01 | 0.40 ± 0.1 |
| **pMCV1.4 G** | 0.60 ± 0.1 | 0.16 ± 0.1 | 0.65 ± 0.1 |
| **pT2 pMCV1.4 G** | 0.95 ± 0.15 | 0.32 ± 0.15 | 0.70 ± 0.2 |

Means ± standard deviations of 4 trout are represented

### REFERENCES

Capriglione, T., Odierna G., Caputo, V., Canapa, A. and Olmo, E. (2002). Characterization of a Tc1-like transposon in the Antartic ice-fish, Chionodraco hamatus. Gene 295, 193-198.
Coll, J.M. (2001). El transposón SB de salmónidos como vector para transferencia de genes en vertebrados. Investigaciones Agrarias 16, 237-244.
Cui, Z., Geurts, A.M., Liu, G., Kaufman, D. and Hackett, P.B. (2002). Structure-function analysis of the inverted terminal repeats of the sleeping beauty transposon. Journal Molecular Biology 318, 1221-1235.
Davidson, A.E., Balciunas, D., Mohn, D., Shaffer, J., Hermanson, S., Sivasubbu, S., Cliff, M.P., Hackett, P.B. and Ekker, C. (2003). Efficient gene delivery and gene expression in zebrafish using the sleeping beauty transposon. Developmental Biology 263, 191-202.
Dupuy, A.J., Clark, K., Carlson, C.M., Fritz, S., Davidson, A.E., Markley, K.M., Finley, K., Fletcher, C.F., Ekker, S.C., Hackett, P.B., Hom, S. and Largaespeda, D.A. (2002). Mammalian germ-line transgenesis by transposition. Proceedings National Academy Sciences USA 99, 4495-4499.
Estepa, A., DeBlas, C., Ponz, F. and Coll, J.M. (1995). Diagnosis of trout haemorrhagic septicaemia rhabdovirus by capture with monoclonal antibodies and amplification with PCR. Veterinary Research 26, 530-532.
Estepa, A., Rocha, A., Pérez, L., Encinar, J.A., Núñez, E., Fernández, A., González Ros, J.M., Gavilanes, F. and Coll, J.M. (2001). A protein fragment from the salmonid VHS rhabdovirus induces cell-to-cell fusion and membrane phosphatidylserine translocation at low pH. Journal Biological Chemistry 276, 46268-46275.
Estepa, A., Thiry, M. and Coll, J.M. (1994). Recombinant protein fragments from haemorrhagic septicaemia rhabdovirus stimulate trout leucocyte anamnestic in vitro responses. Journal General Virology 75, 1329-1338.
Fernández-Alonso, M., Álvarez, F., Estepa, A., Blasco, R. and Coll, J.M. (1999). A model to study fish DNA immersion-vaccination by using the green fluorescent protein. Journal Fish Diseases 22, 237-241.
Fernández-Alonso, M., Rocha, A. and Coll, J.M. (2001). DNA vaccination by immersion and ultrasound to trout viral haemorrhagic septicaemia virus. Vaccine 19, 3067-3075.
Geurts, A.M., Yang, Y., Clark, K.J., Liu, G., Cui, Z., Dupuy, A.J., Bell, J.B., Largaespada, D.A. & Hackett, P.B. (2003). Gene transfer into genomes of human cells by the sleeping beauty transposon system. Molecular Therapy 8, 108-117.
Goodier, J.L. and Davidson, S.D. (1994). Tc1 transposon-like sequences are widely distributed in salmonids. Journal Molecular Biology 241, 26-34.
Gottgens, B., Barton, L.M., Grafham, D., Vaudin, M. and Green, A.R. (1999). Tdr2, a new zebrafish transposon of the Tc1 family. Gene 239, 373-379.
Grabher, C., Henrich, T., Sasado, T., Arenz, A., Wittbrodt, J. and Furutani-Seiki, M. (2003). Transposon-mediated enhancer trapping in medaka. Gene 322, 57-66.
Harris, J.W., Strong, D.D., Amoui, M., Baylink, D.J. and Lau, K.H.W. (2002). Construction of a Tc1-like transposon Sleeping beauty-based gene transfer plasmid vector for generation of stable transgenic mammalian cell clones. Analytical Biochemistry 310, 15-26.
Heierhorst, J., Lederis, K. and Richter, D. (1992). Presence of a member of the Tc1-like transposon family from nematodes and Drosophila within the vasotocin gene of a primitive vertebrate, the Pacific hagfish Eptatretus stouti. Proceedings National Academy Sciences USA 89, 6798-6802.
Henikoff, J. (1992). Detection of Caenorhabditis transposon homologs in diverse organisms. New Biologist 4, 382-388.
Horie, K., Yusa, K., Yae, K., Odajima J., Fisher, S.E., Keng, V.W., Hayakawa, T., Mizuno, A., Kondoh, G., Ijiri, T., Matsuda, Y., Plasterk, R.H. and Takeda, J. (2003). Characterization of sleeping beauty transposition and its application to genetic screening in mice. Molecular Cell Biology 23, 9189-9207.
Iida, A., Tachibana A., Hamada S., Hori, H. and Koga, A. (2004). Low transposition frequency of the medaka fish To12 element may be due to extranuclear localization of its transposase. Genes Genetic Syst 79, 119-124.
Ivics, Z. and Izsvak, Z. (2002). Transposable elements for transgenesis and insertional mutagenesis in vertebrates. A contemporary review of experimental strategies. Methods in Molecular Biology 260, 255-276.
Ivics, Z., Izsvak, Z. and Hackett, P.B. (1997). Molecular reconstruction of sleeping beauty, a Tc1-like transposon system from fish and its transposition in human cells. Cell 91, 501-510.
Ivics, Z., Izsvak, Z., Minter, A. and Hackett, P. (1996a). Identification of functional domains and evolution of Tc1-like transposable elements. Proceedings National Academy Sciences USA 93, 5008-5013.
Ivics, Z., Izsvak, Z., Minter, A. and Hackett, P.B. (1996b). Identification of functional domains and evolution of Tc1-like transposable elements. Proceedings National Academy Sciences USA 93, 5008-5013.
Izsvak, Z. and Ivics, Z. (2003). Sleeping beauty transposition: Biology and applications for molecular therapy. Molecular Therapy 9, 147-156.
Izsvak, Z., Ivics, Z. and Hackett, P.B. (1995). Characterization of a Tc1-like transposable element in zebrafish (Danio rerio). Molecular General Genetics 247, 312-322.
Izsvak, Z., Ivics, Z. and Hackett, P.B. (1997). Repetitive elements and their genetic applications in zebrafish. Biochemical Cell Biology 75, 507-523.
Izsvak, Z., Ivics, Z. and Plasterk, R.H. (2000). Sleeping beauty, a wide host-range transposon vector for genetic transformation in vertebrates. Journal Molecular Biology 302, 93-102.
Izsvak, Z., Khare, D., Behlke, J., Heinemann, U., Plasterk, R.H. and Ivics, Z. (2002). Involvement of a bifunctional, paired like DNA-binding domain and a transpositional enhancer in sleeping beauty transposition. Journal Biological Chemistry 277, 34581-34588.
Kawakami, K., Imanaka, K., Itoh, M. and Taira, M. (2004). Excision of the To12 transposable element of the medaka fish Oryzias latipes in Xenopus laevis and Xenopus tropicalis. Gene 338, 93-98.
Kawakami, K. and Shima, A. (1999). Identification of the To12 transposase of the medaka fish Oryzias latipes that catalyzes excision of a nonautonomous Tol2 element in zebrafish Danio rerio. Gene 240, 239-244.
Kido, Y., Himberg, M., Takasaki, N. and Okada, N. (1994). Amplification of distinct subfamilies of short interdispersed elements during evolution of the salmonidae. Journal Molecular Biology 241, 633-644.
Koga, A., Suzuki, M., Inagaki, H., Bessho, Y. and Hori, H. (1996). Transposable element in fish. Nature 383, 30.
Lam, W.L., Lee, T.S. and Gilb€rt, W. (1996). Active transposition in zebrafish. Proceedings National Academy Sciences USA 93, 10870-10875.
Lampe, D.J., Churchill, M.E.A. and Robertson, H.M. (1996). A purified mariner transposase is sufficient to mediate transposition in vitro. EMBO Journal 15, 5470-5479.
Leaver, M.J. (2001). A family of Tc1-like transposons from the genomes of fishes and frogs: Evidence for horizontal transmission. Gene 271, 203-214.
López, A., Fernández-Alonso, M., Rocha, A., Estepa, A. and Coll, J.M. (2001). Transfection of epithelioma cyprini (EPC) carp cells. Biotechnology Letters 23, 481-487.
Masuda, K., Yamamoto, S., Endoh, M. and Kaneda, Y. (2004). Transposon-independent increase of transcription by the sleeping beauty transposase. Biochemical Biophysical Research Communications 317, 796-800.
Mikkelsen, J.G., Yant, S.R., Meuse, L., Huang Z., Xu, H. and Kay, M.A. (2003). Helper-independent sleeping beauty transposon-transposase vectors for efficient nonviral gene delivery and persistent gene expression in vivo. Molecular Therapy 8, 654-665.
Plasterk, R.H.A., Izsvak, Z. and Ivics, Z. (1999). Resident Aliens. The Tc1/mariner superfamily of transposable elements. TIG 15, 326-332.
Radice, A.D., Bugaj, B., Fitch, D.H.A. and Emmons, S.W. (1994). Widespread occurrence of the Tc1 transposon family: Tc1-like transposons from teleost fish. Molecular General Genetics 244, 606-612.
Reed, K.M. (1999). Tc1-like transposable elements in the genome of lake trout (Salvelinus namycush). Marine Biotechnology 1, 60-67.
Rocha, A., Ruiz, S. and Coll, J.M. (2004). Improvement of transfection efficiency of epithelioma papulosum cyprini carp cells by modification of their cell cycle and using an optimal promoter. Marine Biotechnology, in press.
Rubinsky, I. and Ruvkun, G. (2003). Functional test of enhancer conservation between distantly related species. Developmental Biology 130, 5133-5142.
Sambrook, J., Fritsch, E.F. and Maniatis, T. (1989). Molecular cloning. A laboratory manual. Cold Spring Harbour Laboratory Press.
Sánchez-Puig, J.M. and Blasco, R. (2000). Puromycin resistance (pac) gene as a selectable marker in vaccinia virus. Gene 257, 57-65.
Shen, C.H. & Steiner, L.A. (2004). Genome structure and thymic expression of an endogenous retrovirus in zebrafish. Journal Virology 78: 899-911.
Tafalla, C., Estepa, A. and Coll, J.M. (2005a). Development and possible applications to aquaculture of sleeping beauty, the first active vertebrate transposon obtained from fish. Aquaculture, submitted.
Tafalla, C., Estepa., A. and Coll, J.M. (2005b). Potential use of fish transposons in Aquaculture. Journal Biotechnology, accepted.
Tafalla, C., Coll, J. and Secombes, C.J. (2005). Expression of genes related to the early immune response in rainbow trout (Oncorhynchus mykiss) after viral haemorrhagic septicemia virus (VHSV) infection. Developmental and Comparative Immunology 29:615-626.
TakaHashi, K., Terai, Y., Nishida, M. and Okada, N. (1998). A novel familiy of short interspersed repetitive elements (SINEs) from cichilds: The patterns of insertion of SINEs at orthologous loci support the proposed monophyly of four major groups of cichild fishes in lake Tanganyika. Molecular Biological Evolution 15, 391-407.
Winkfein, R.J., Moir, R.D., Krawets, J., Blanco, J., States, C. and Dixon, G.H. (1988). A new family of repetitive retroposon-like sequences in the genomes of the rainbow trout. European Journal Biochemistry 176, 255-264.
Yant, S.R., Meuse, L., Chiu, W., Ivics, Z., Izsvak, Z. and Kay, M.A. (2000). Somatic integration and longterm transgene expresion in normal and haemophilic mice using a DNA transposon system. Nature Genetics 25, 35-41 .
Yant, S.R., Park, J., Huang, Y., Mikkelsen, J.G. and Kay, M. A. (2004). Mutational analysis of the N-terminal DNA-binding domain of sleeping beauty transposase: Critical residues for DNA binding and hyperactivity in mammalian cells. Molecular Cell Biology 24, 9239-9247.
Yusa, K., Takeda, J. and Horie, K. (2004). Enhancement of sleeping beauty transposition by CpG methylation: Possible role of heterochromatin formation. Molecular Cell Biology 24, 4004-4018.
Zayed, H., Izsvak, Z., Khare, D., Heinemann, U. and Ivics, Z. (2003). The DNA-bending protein HMGB1 is a cellular cofactor of sleeping beauty transposition. Nucleic Acids Research 31, 2313-2322.
Zayed, H., Izsvak, Z., Walisko, O. and Ivics, Z. (2004). Development of hyperactive sleeping beauty transposon vectors by mutational analysis. Molecular Therapy 9, 292-304.
Zhang, G., Hiraiwa, H., Yasue, H., Wu, H., Ross, C.R., Troyer, D. and Blecha, F. (1999). Cloning and characterization of the gene for a new epithelial β-defensin. Journal Biological Chemistry 274: 24031-24037.

## Claims

1. A gene construct that comprises an expression control sequence capable of directing the expression of an immunogenic peptide in an aquatic animal, and a nucleic acid sequence that encodes an immunogenic peptide of an aquatic animal pathogen, where said expression control sequence is a nucleotide sequence that comprises an A sequence and a B sequence, where said A sequence comprises an expression enhancer sequence, and said B sequence comprises the cytomegalovirus (CMV) promoter sequence, and where the 3' end of said A sequence is bound to the 5' end of said B sequence.

2. Construct, according to claim 1, wherein said A sequence is composed of the 218-base sequence between nucleotide 1 and nucleotide 218 of the nucleotide sequence shown in SEQ ID NO:1.

3. Construct, according to claim 1, wherein said B sequence is composed of the CMV promoter sequence.

4. Construct, according to claim 1, wherein said expression control sequence comprises the nucleotide sequence shown in SEQ ID NO:1.

5. Construct, according to claim 1, wherein said immunogenic peptide of an aquatic animal pathogen is selected from complete proteins and fragments thereof, and peptide epitopes associated with viruses, bacteria or parasites that cause diseases in aquatic animals.

6. Construct, according to claim 5, wherein said immunogenic peptide of an aquatic animal pathogen is selected from protein G (pG) of the haemorrhagic septicaemia virus of trout (VHSV); pG of the infectious haematopoietic necrosis virus (IHNV); proteins VP1, VP2, VP3 or the structural N protein of the infectious pancreatic necrosis virus (IPNV); pG of spring viremia of carp (SVC); protein p57 of *Renibacterium salmoninarum;* and surface antigens of *Ichthyophthirius.*

7. Construct, according to claim 1, that comprises two or more nucleic acid sequences that encode two or more immunogenic peptides of one or more aquatic animal pathogens.

8. A vector that comprises a gene construct, according to any of claims 1 to 7.

9. Vector, according to claim 8, where said vector is a transposon.

10. Transposon that comprises a gene construct, according to any of claims 1-7, where said construct has had the cytomegalovirus promoter intron deleted.

11. Use of a transposon, according to claim 10, for the preparation of a vaccine designed to confer protection to aquatic animals against aquatic animal pathogens.

12. Use of a transposon, according to claim 10, in the preparation of a vaccine for administration to aquatic animals by a process that comprises the immersion of said aquatic animals in a bath containing said vaccine and the sonication of said bath containing said aquatic animals and said vaccine.

13. Vaccine that comprises a transposon, according to claim 10, and, optionally, one or more pharmaceutically acceptable adjuvants and/or vehicles.

14. Vaccine, according to claim 13, where said vaccine is a DNA vaccine.

15. Vaccine, according to any of claims 13-14, designed to protect aquatic animals susceptible to being infected by aquatic animal pathogens selected from the haemorrhagic septicaemia virus of trout (VHSV), the haematopoietic necrosis virus (IHNV), the infectious pancreatic necrosis virus (IPNV), the virus that causes spring viremia of carp (SVCV), *Aeromonis salmonicida, Renibacterium salmoninarum, Yersinia, Pasteurella, Vibrosis, Edwardsiella, Streptococci* and *Ichthyophthirius.*
